# EUROPEAN PATENT APPLICATION

(11) **EP 1 685 830 A1**
(43) Date of publication of application: **02.08.2006**
(21) Application number: 04819003.7
(22) Date of filing: 19.11.2004
(51) Int. Cl.: A61K 31/137, A61K 9/70, A61K 47/10, A61K 47/32, A61P 11/00, A61P 11/06, A61P 11/08

(54) **PLASTER**

(30) Priority: 21.11.2003 JP 2003392914
(71) Applicant: SEKISUI CHEMICAL CO., LTD., Osaka-shi, Osaka 530-8565 (JP)
(72) Inventor: Udagawa, Hiroko, c/o Sekisui Chemical Co., Ltd., Mishima-gun, Osaka 6280021 (JP); Komoda, Toshikazu, c/o Sekisui Chemical Co., Ltd., Mishima-gun, Osaka 6180021 (JP); Hamabe, Masaru, c/o Sekisui Chemical Co., Ltd., Mishima-gun, Osaka 6180021 (JP)
(74) Representative: Merkle, Gebhard
(86) International application number: PCT/JP2004/017305
(87) International publication number: WO 2005/049004

(57) **Abstract**

The present invention provides an adhesive skin patch that permits excellent and prolonged percutaneous absorption of tulobuterol and that can be stably applied to the skin for a long period of time with less skin irritation. The adhesive skin patch comprises a base material and a medicated layer provided on one surface of the base material. The medicated layer contains 1 to 30% by weight of a resolvent, 40 to 98% by weight of a pressure-sensitive adhesive, and tulobuterol. The resolvent contains an aliphatic alcohol having a branched-chain structure or a double bond in its C8 to C30 carbon chain. The pressure-sensitive adhesive is a copolymer obtained by copolymerizing monomers containing 70% by weight or more of an alkyl(meth)acrylate whose alkyl group has 6 to 20 carbon atoms.

## Description

### TECHNICAL FIELD

The present invention relates to a tulobuterol percutaneous absorption preparation, more particularly, to an adhesive skin patch that permits prolonged percutaneous absorption of tulobuterol and that can be stably applied to the skin for a long period of time with less skin irritation.

### BACKGROUND ART

Tulobuterol selectively stimulates β₂ receptors on bronchial smooth muscle, and is therefore used as a bronchodilator. More specifically, tulobuterol is widely used to relieve dyspnea associated with airway obstruction and to treat bronchial asthma, acute bronchitis, and chronic bronchitis that become the cause of an attack of airway obstruction.

Tulobuterol is generally administered to a living body by oral administration using tablets or by percutaneous administration using adhesive skin patches. In recent years, the latter method using adhesive skin patches is receiving attention because it is possible to administer a desired dose of tulobuterol to infants, and to prevent side effects resulting from a rapid increase in drug level in the blood, and to sustain drug efficacy so that dyspnea is effectively prevented when airway obstruction occurs.

Generally, when a drug is administered to a living body using an adhesive skin patch, the drug is transferred to the skin from the adhesive skin patch and is then absorbed through the skin based on a concentration gradient between the drug concentration in a medicated layer of the adhesive skin patch and the drug concentration in the skin. Therefore, by dissolving the drug in the medicated layer of the adhesive skin patch at a concentration closer to its saturation solubility in the medicated layer, it is possible to further improve percutaneous absorption of the drug.

Adhesive skin patches are broadly divided into two types: those in which part of a drug is deposited in a crystalline state on the surface of a medicated layer; and those in which a drug is entirely dissolved in a medicated layer. In a case where the former adhesive skin patch is used, the concentration of the drug in the medicated layer can be made equal to its saturation solubility in the medicated layer while the drug deposited on the surface of the medicated layer is gradually dissolved in the medicated layer. This is advantageous to percutaneous absorption of the drug. Such adhesive skin patches have already been disclosed in Patent Documents 1 and 2.

However, there is a case where the drug deposited on the surface of the medicated layer is once dissolved in the medicated layer but is recrystallized depending on the storage conditions of the adhesive skin patches. In this case, there is a possibility that the crystal size or crystal type of the drug is changed, thereby causing a problem that percutaneous absorption of the drug varies. Further, in a case where the drug is gradually deposited on the surface of the medicated layer after the adhesive skin patch is manufactured, there is a problem that adhesion of the adhesive skin patch to the skin varies with the lapse of time. Furthermore, in a case where a large amount of the drug is recrystallized on the surface of the medicated layer, there is a fear that the rate of percutaneous absorption of the drug becomes too high just after the adhesive skin patch is applied to the skin so that it is impossible to keep the rate of percutaneous absorption constant.

On the other hand, in a case where the latter adhesive skin patch in which a drug is entirely dissolved in a medicated layer is used, the drug concentration in the medicated layer is decreased as the drug contained in the medicated layer is absorbed through the skin. If the solubility of the drug in the medicated layer is low, a concentration gradient between the drug concentration in the medicated layer and the drug concentration in the skin becomes small in a short period of time, thereby causing a problem that percutaneous absorption of the drug cannot be maintained for a long period of time.

In order to solve such a problem, Patent Document 3 has disclosed an adhesive skin patch obtained by providing, on a base material, a medicated layer comprising a resolvent such as alcohols, a pressure-sensitive adhesive composed of an acrylic ester-acrylic acid copolymer, and tulobuterol.

However, the solubility of the drug in the medicated layer depends on temperature, and therefore even when the drug is dissolved in the medicated layer at a high concentration equal to its saturation solubility in the medicated layer, there is a case where the solubility of the drug in the medicated layer is significantly reduced due to environmental changes such as changes in seasons or places so that the drug dissolved in the medicated layer is crystallized and the crystalline drug is deposited on the surface of the medicated layer. If the drug is deposited on the surface of the medicated layer, there is a problem that percutaneous absorption of the drug is lower than originally intended so that therapeutic action is adversely affected.

Further, if the concentration of the resolvent in the medicated layer is too high, there is a problem that an adhesive residue remains on the skin after the adhesive skin patch is peeled off from the skin or, inversely, the adhesive skin patch is easily peeled off from the skin. In addition, there is also a problem that the initial rate of percutaneous absorption of the drug becomes too high to stably maintain a desired rate of percutaneous absorption.

Patent Document 1: Japanese Patent No. 3260765
Patent Document 2: Japanese Patent No. 2753800
Patent Document 3: Japanese Unexamined Patent Publication No. 63-10716

### DISCLOSURE OF THE INVENTION

### PROBLEMS TO BE SOLVED BY THE INVENTION

It is therefore an object of the present invention to provide an adhesive skin patch that permits prolonged and excellent percutaneous absorption of tulobuterol and that can be stably applied to the skin for a long period of time with less skin irritation.

### MEANS FOR SOLVING THE PROBLEMS

An adhesive skin patch A according to the present invention comprises a base material B and a medicated layer C provided on one surface of the base material B so as to be integral with the base material B. The medicated layer C contains 100 parts by weight of a pressure-sensitive adhesive, 1 to 75 parts by weight of a resolvent, and tulobuterol. The resolvent contains an aliphatic alcohol having a branched-chain structure or a double bond in its C8 to C30 carbon chain, and the pressure-sensitive adhesive is a copolymer obtained by copolymerizing monomers containing 70% by weight or more of an alkyl(meth)acrylate whose alkyl group has 6 to 20 carbon atoms.

As described above, the pressure-sensitive adhesive constituting the medicated layer C comprises a copolymer obtained by copolymerizing monomers containing 70% by weight or more of an alkyl(meth)acrylate whose alkyl group has 6 to 20 carbon atoms. Examples of such an alkyl(meth)acrylate whose alkyl group has 6 to 20 carbon atoms include, but are not limited to, hexyl acrylate, hexyl methacrylate, octyl acrylate, octyl methacrylate, decyl acrylate, decyl methacrylate, dodecyl acrylate, dodecyl methacrylate, tridecyl acrylate, tridecyl methacrylate, octadecyl acrylate, octadecyl methacrylate, 2-ethylhexyl acrylate, 2-ethylhexyl methacrylate, and the like.

Preferred examples of the pressure-sensitive adhesive include copolymers obtained by copolymerizing monomers containing 2-ethylhexyl(meth)acrylate and an alkyl(meth)acrylate whose alkyl group is linear and has 6 to 20 carbon atoms. By using such a pressure-sensitive adhesive together with the resolvent, it is possible to impart excellent tulobuterol-releasing characteristics and skin adhesion properties to the medicated layer. It is to be noted that in this specification, (meth)acryl means methacryl and acryl.

If the amount of 2-ethylhexyl(meth)acrylate contained in monomers containing 2-ethylhexyl(meth)acrylate and an alkyl(meth)acrylate whose alkyl group is linear and has 6 to 20 carbon atoms is too small, there is a case where the adhesive strength of the pressure-sensitive adhesive is low. On the other hand, if the amount is too large, there is a case where elasticity and cohesion of the pressure-sensitive adhesive are low and therefore an adhesive residue remains on the skin after the adhesive skin patch is peeled off from the skin. For this reason, the amount is preferably 70 to 95% by weight, more preferably 70.5 to 95% by weight.

For the similar reason to above, the amount of an alkyl(meth)acrylate component whose alkyl group is linear and has 6 to 20 carbon atoms contained in monomers containing 2-ethylhexyl(meth)acrylate and an alkyl(meth)acrylate whose alkyl group is linear and has 6 to 20 carbon atoms is preferably 5 to 30% by weight, more preferably 5 to 29.5% by weight.

Further, monomers for obtaining a copolymer constituting the pressure-sensitive adhesive may contain a monomer such as an alkyl(meth)acrylate whose alkyl group has 5 or less carbon atoms (e.g., methyl acrylate, methyl methacrylate, ethyl acrylate, ethyl methacrylate, butyl acrylate, butyl methacrylate), acrylic acid, methacrylic acid, vinyl pyrrolidone, vinyl acetate or hydroxyethyl acrylate, as long as tulobuterol-releasing characteristics and stability of tulobuterol are not impaired.

Furthermore, monomers for obtaining a copolymer constituting the pressure-sensitive adhesive may contain a multifunctional monomer to obtain a crosslinked copolymer. By doing so, it is possible to prevent an adhesive residue from remaining on the skin after the adhesive skin patch A is peeled off from the skin. Examples of such a multifunctional monomer include, but are not limited to, di(meth)acrylates, tri(meth)acrylates, and tetra(meth)acrylates that are obtained by reacting (meth)acrylic acid and a polyalkylene glycol such as polymethylene glycol (e.g., hexamethylene glycol), glycerol or pentaerythritol. Among them, hexanediol di(meth)acrylate is preferably used.

If the amount of a multifunctional monomer contained in monomers for obtaining a copolymer constituting the pressure-sensitive adhesive is too small, there is a case where the above-described effect obtained by using the multifunctional monomer is not exhibited. On the other hand, if the amount is too large, the pressure-sensitive adhesive is easily turned into a gel. For this reason, the amount is preferably 0.005 to 0.5% by weight.

More specifically, in a case where monomers for obtaining a copolymer constituting the pressure-sensitive adhesive contain a multifunctional monomer, the pressure-sensitive adhesive is preferably a copolymer obtained by copolymerizing monomers containing 2-ethylhexyl(meth)acrylate, an alkyl(meth)acrylate whose alkyl group is linear and has 6 to 20 carbon atoms, and a multifunctional monomer.

If the amount of 2-ethylhexyl(meth)acrylate contained in monomers containing 2-ethylhexyl(meth)acrylate, an alkyl(meth)acrylate whose alkyl group is linear and has 6 to 20 carbon atoms, and a multifunctional monomer is too small, there is a case where the adhesive strength of the pressure-sensitive adhesive is low. On the other hand, if the amount is too large, there is a case where elasticity and cohesion of the pressure-sensitive adhesive are low and therefore an adhesive residue remains on the skin after the adhesive skin patch is peeled off from the skin. For this reason, the amount is preferably 70 to 94.5% by weight.

Further, if the amount of an alkyl(meth)acrylate, whose alkyl group is linear and has 6 to 20 carbon atoms, contained in monomers containing 2-ethylhexyl(meth)acrylate, an alkyl(meth)acrylate whose alkyl group is linear and has 6 to 20 carbon atoms, and a multifunctional monomer is too small, there is a case where elasticity and cohesion of the pressure-sensitive adhesive are low and therefore an adhesive residue remains on the skin after the adhesive skin patch is peeled off from the skin. On the other hand, if the amount is too large, there is a case where the adhesive strength of the pressure-sensitive adhesive is low. For this reason, the amount is preferably 5 to 29.5% by weight.

Furthermore, if the amount of a multifunctional monomer component contained in monomers containing 2-ethylhexyl(meth)acrylate, an alkyl(meth)acrylate whose alkyl group is linear and has 6 to 20 carbon atoms, and a multifunctional monomer is too small, there is a case where the above-described effect obtained by using the multifunctional monomer is not exhibited. On the other hand, if the amount is too large, the pressure-sensitive adhesive is easily turned into a gel. For this reason, the amount is preferably 0.005 to 0.5% by weight.

The pressure-sensitive adhesive may contain a crosslinking agent, as long as the stability of tulobuterol is not adversely affected. By adding a crosslinking agent to the pressure-sensitive adhesive, it is possible to improve cohesion of the pressure-sensitive adhesive and to reduce an adhesive residue, thereby enabling an adhesive skin patch excellent in skin adhesion properties to be obtained. Examples of such a crosslinking agent include epoxy compounds, polyisocyanate compounds, metal chelate compounds, metal alkoxide compounds, and the like.

Next, a method for producing the pressure-sensitive adhesive will be described. The pressure-sensitive adhesive can be produced by a method generally used, such as solution polymerization. More specifically, predetermined amounts of an alkyl(meth)acrylate whose alkyl group has 6 to 20 carbon atoms and a polymerization initiator, and if necessary, a multifunctional monomer, another monomer, and a crosslinking agent are fed, together with a solvent for polymerization, into a reactor equipped with a stirrer and a reflux condenser for a vaporized solvent, and then they are heated to, for example, about 80°C for 8 to 40 hours to carry out radical polymerization of the alkyl(meth)acrylate. It is to be noted that predetermined amounts of the alkyl(meth)acrylate, the solvent for polymerization, and the polymerization initiator may be fed into the reactor at a time or over several times. As a solvent for polymerization, a solvent generally used for polymerization, such as ethyl acetate can be used. Polymerization is preferably carried out in an atmosphere of nitrogen gas.

The polymerization initiator is not particularly limited as long as it is a conventionally used initiator. Examples of such a polymerization initiator include: azobis-type polymerization initiators such as 2,2'-azobis-isobutyronitrile (AIBN), 1,1'-azobis(cyclohexane-1-carbonitrile), and 2,2'-azobis(2,4-dimethylvaleronitrile); and peroxide-based polymerization initiators such as benzoyl peroxide (BPO), lauroyl peroxide (LPO), and di-tert-butyl peroxide.

As described above, the resolvent contained in the medicated layer C comprises an aliphatic alcohol, and the aliphatic alcohol is not particularly limited as long as it has a branched-chain structure or a double bond in its C8 to C30 carbon chain. Examples of such an aliphatic alcohol include: aliphatic alcohols having a branched-chain structure, such as ethyl hexanol, hexyl decanol, octyl dodecanol, and isostearyl alcohol; and aliphatic alcohols having a double bond, such as oleyl alcohol, linoleyl alcohol, and elaidyl alcohol. Among them, primary aliphatic alcohols having a branched-chain structure or a double bond in its C8 to C30 carbon chain are preferred, and primary aliphatic alcohols having a branched-chain structure in its C8 to C30 carbon chain are more preferred. Particularly, 2-octyl-1-dodecanol is preferred. These aliphatic alcohols may be used singly or in combination of two or more of them.

If the number of carbon atoms contained in the carbon chain of the aliphatic alcohol is too small, volatility of the aliphatic alcohol is high and mutual solubility with a copolymer constituting the pressure-sensitive adhesive is poor. On the other hand, if the number is too large, solubility of tulobuterol is decreased so that it becomes impossible to dissolve tulobuterol in the medicated layer to a desired concentration. For this reason, the number of carbon atoms contained in the carbon chain of the aliphatic alcohol is limited to 8 to 30, preferably 12 to 24.

If the amount of an aliphatic alcohol, having a branched-chain structure or a double bond in its C8 to C30 carbon chain, contained in the medicated layer C is too small, solubility of tulobuterol in the medicated layer is decreased. On the other hand, if the amount is too large, there is a problem that an adhesive residue remains on the skin after the adhesive skin patch is peeled off from the skin or, inversely, the adhesive skin patch is easily peeled off from the skin. In addition, there is another problem that the initial rate of percutaneous absorption of the drug becomes too high to fail to stably maintain a desired rate of percutaneous absorption. For this reason, the amount is limited to 1 to 75 parts by weight, preferably 1 to 40 parts by weight, more preferably 2 to 40 parts by weight, particularly preferably 2 to 30 parts by weight, with respect to 100 parts by weight of the pressure-sensitive adhesive.

The amount of tulobuterol contained in the medicated layer C is not particularly limited as long as the rate of percutaneous absorption is high enough to exhibit drug efficacy and adhesion of the medicated layer to the skin is not impaired. More specifically, if the amount of tulobuterol contained in the medicated layer is too small, there is a case where it is impossible to keep a desired rate of percutaneous absorption of tulobuterol so that a desired concentration of tulobuterol in the blood cannot be achieved. On the other hand, if the amount is too large, there is a case where tulobuterol has a plasticizing effect on the pressure-sensitive adhesive so that the adhesive strength of the medicated layer is increased, which is likely to cause skin irritation when the adhesive skin patch is peeled off from the skin. Further, there is also a case where the utilization ratio of tulobuterol is decreased, which is disadvantageous from the viewpoint of efficiency. For this reason, the amount is preferably 0.5 to 75 parts by weight, more preferably 1 to 25 parts by weight, particularly preferably 2 to 11 parts by weight, with respect to 100 parts by weight of the pressure-sensitive adhesive.

If necessary, the medicated layer C may further contain a plasticizer, a percutaneous absorption accelerator, a stabilizer, a filler and the like. By adding a plasticizer to the medicated layer C, it is possible to control the adhesion of the medicated layer to the skin. Some plasticizers also have the effect of increasing the diffusion rate of tulobuterol in the medicated layer so that the amount of tulobuterol absorbed through the skin is increased. Examples of such a plasticizer include: hydrocarbons such as liquid paraffin; esters of fatty acids and monovalent or polyvalent alcohols, such as isopropyl myristate, glycerol monolaurate, and diethyl sebacate; and naturally-derived oils and fats such as lanolin and olive oil.

If the amount of a plasticizer contained in the medicated layer C is too small, there is a case where the above-described effect obtained by adding a plasticizer to the medicated layer is not exhibited. On the other hand, if the amount is too large, there is a case where the solubility of tulobuterol in the medicated layer C is decreased or the adhesion of the adhesive skin patch to the skin becomes poor. For this reason, the amount is preferably 1 to 15% by weight.

When a percutaneous absorption accelerator is added to the medicated layer, the percutaneous absorption accelerator acts on the skin to improve the skin permeability of tulobuterol. For example, addition of a percutaneous absorption accelerator has the effect of relaxing the structure of the stratum corneum or improving the hydration of the stratum corneum. Examples of such a percutaneous absorption accelerator include: surfactants such as polysorbate, lauric acid diethanol amide, lauroyl sarcosine, polyoxyethylene alkyl ethers and polyoxyethylene alkyl amines; and polyvalent alcohols such as polyethylene glycol and glycerol.

If the amount of a percutaneous absorption accelerator contained in the medicated layer C is too small, there is a case where the above-described effect obtained by adding the percutaneous absorption accelerator to the medicated layer is not exhibited. On the other hand, if the amount is too large, there is a case where the solubility of tulobuterol in the medicated layer and the stability of tulobuterol are adversely affected. For this reason, the amount is preferably 0.1 to 10% by weight.

When a stabilizer is added to the medicated layer, the stabilizer suppresses oxidation and decomposition of tulobuterol and other components to prevent deterioration of the adhesive skin patch with the lapse of time. Examples of such a stabilizer include antioxidants such as butylated hydroxy toluene and sorbic acid; cyclodextrin; ethylenediaminetetraacetic acid, and the like.

If the amount of a stabilizer contained in the medicated layer C is too small, there is a case where the above-described effect obtained by adding the stabilizer to the medicated layer C is not exhibited. On the other hand, if the amount is too large, there is a case where adhesion of the adhesive skin patch to the skin is adversely affected or skin irritation is caused. For this reason, the amount is preferably 0.05 to 10% by weight.

When a filler is added to the medicated layer, the filler controls the adhesion of the medicated layer C to the skin and tulobuterol-releasing characteristics. Examples of such a filler include calcium carbonate, titanium oxide, lactose, crystalline cellulose, silicic acid anhydride, and the like.

If the amount of a filler contained in the medicated layer C is too small, there is a case where the above-described effect obtained by adding a filler to the medicated layer C is not exhibited. On the other hand, if the amount is too large, there is a case where adhesion of the adhesive skin patch to the skin contrarily becomes poor, or percutaneous absorption of tulobuterol and stability of tulobuterol are adversely affected. For this reason, the amount is preferably 1 to 30% by weight.

The base material B provided so as to be integral with the medicated layer C is flexible but has the function of imparting self supportability to the adhesive skin patch and preventing a loss of tulobuterol contained in the medicated layer. Examples of such a material for the base material B include polyesters such as polyethylene terephthalate and nylon, cellulose acetate, ethyl cellulose, rayon, plasticized vinyl acetate-vinyl chloride copolymers, plasticized polyvinyl chloride, polyurethane, polyethylene, ethylene-vinyl acetate copolymers, ethylene-methyl (meth)acrylate copolymers, polyvinylidene chloride, aluminum, polyvinyl alcohol, SIS copolymers, SEBS copolymers, cotton, and the like. Among these materials, polyethylene terephthalate is preferably used.

The form of the base material B is not particularly limited. Examples of the form of the base material B include films, foam sheets, and fabrics such as non-woven fabrics, woven fabrics and knitted fabrics, and they may be used as a single layer or a laminated body obtained by integrally laminating two or more layers made of different materials. Particularly, from the viewpoint of flexibility and drug loss prevention, a laminated film obtained by integrally laminating a flexible resin film made of, for example, an ethylene-vinyl acetate copolymer or a non-woven fabric and a polyethylene terephthalate film is preferably used. It is to be noted that a method for integrally laminating two or more layers made of different materials is not particularly limited. For example, a method using an adhesive, a heat-seal method, and a method using a binder can be mentioned.

If the thickness of the polyethylene terephthalate film of the laminated film is too thin, there is a case where the polyethylene terephthalate film is not uniformly adhered to the non-woven fabric or the other resin film when they are integrally laminated so that a pin hole is produced in the polyethylene terephthalate film, or delamination occurs between the non-woven fabric or the resin film and the polyethylene terephthalate film. On the other hand, if the thickness is too thick, the base material B is excessively hard so that the adhesive skin patch cannot smoothly follow the movement of the skin, which is likely to give uncomfortable feeling to users. For this reason, the thickness is preferably 5 to 200 µm.

If the weight per unit area of the non-woven fabric of the laminated film is too small, there is a case where the elasticity of the base material is insufficient so that the handleability of the adhesive skin patch becomes poor. On the other hand, if the weight per unit area is too large, the base material B is excessively hard so that the adhesive skin patch cannot smoothly follow the movement of the skin, which is likely to give uncomfortable feeling to users. For this reason, the weight per unit is preferably 10 to 300 g/m².

The surface of the base material B on which the medicated layer C is to be integrally laminated may be subjected to corona treatment or plasma discharge treatment or may be coated with an anchor coating agent, for the purpose of improving integration of the base material B and the medicated layer C.

On the surface of the medicated layer C of the adhesive skin patch A, a release liner may be releasably laminated to protect the medicated layer C until the time of use. Examples of such a release liner include, but are not limited to, films having one silicone-treated surface. Examples of such a film include: polyester films such as a polyethylene terephthalate film; polyvinyl chloride-based films such as a polyvinyl chloride film and a polyvinylidene chloride film; polyolefin-based films such as a polyethylene film and a polypropylene film; paper such as high-quality paper and glassine paper; laminated films obtained by laminating such paper and polyolefin-based films; paper impregnated with polyvinyl alcohol; and films obtained by integrally providing an aluminum foil or an aluminum evaporation layer on the surface of these films. It is to be noted that the thickness of the release liner is preferably 1 mm or less, more preferably 30 to 200 µm.

Next, a method for manufacturing the adhesive skin patch A will be described. The method for manufacturing the adhesive skin patch A is not particularly limited. For example, a resolvent and a pressure-sensitive adhesive that have been produced in such a manner described above and tulobuterol are completely dissolved in an organic solvent such as ethyl acetate, hexane or toluene to obtain a solution. The solution is applied onto one surface of a base material, and is then dried to remove the solvent. In this way, a medicated layer is laminated on one surface of the base material so as to be integral with the base material, to thereby obtain an adhesive skin patch. Alternatively, the solution obtained by completely dissolving the resolvent, the pressure-sensitive adhesive and tulobuterol in the organic solvent mentioned above may be applied onto one surface of a release liner. The solution is then dried to remove the solvent and as a result, a medicated layer is formed. Then, a base material is integrally laminated on the medicated layer to obtain an adhesive skin patch.

At this time, it is preferred that the medicated layer C of the adhesive skin patch A is formed so as to have a thickness of 10 to 500 µm. If the thickness of the medicated layer C is too small, there is a case where adhesion of the adhesive skin patch to the skin becomes poor. On the other hand, if the thickness is too large, the adhesive strength of the adhesive skin patch is too strong so that users have a skin irritation when the adhesive skin patch is peeled off from the skin.

### EFFECTS OF THE INVENTION

According to the adhesive skin patch of the present invention, since specific resolvent and pressure-sensitive adhesive are used together, it is possible to stably dissolve tulobuterol in the medicated layer within a wide temperature range and at a concentration that cannot be achieved by a conventional method. Therefore, tulobuterol can be effectively absorbed through the skin at a desired rate of percutaneous absorption.

Further, the resolvent used in the adhesive skin patch of the present invention allows tulobuterol to well dissolve in the medicated layer. In addition, the resolvent itself not only serves as a carrier for carrying tulobuterol into the skin but also plasticizes the pressure-sensitive adhesive. That is, the resolvent has the function of increasing the diffusion rate of tulobuterol in the pressure-sensitive adhesive and encouraging the absorption rate of tulobuterol through the skin.

Therefore, the adhesive skin patch of the present invention can maintain an appropriate rate of percutaneous absorption of tulobuterol for a long period of time even in a case where the tulobuterol concentration in the medicated layer is relatively low or the tulobuterol concentration in the medicated layer is decreased with the lapse of time during use. Further, although tulobuterol is present in a dissolved state in the medicated layer, tulobuterol is utilized with a high degree of efficiency. For this reason, the medicated layer does not need to contain a high concentration of tulobuterol, and therefore it is possible to prevent plasticization of the pressure-sensitive adhesive caused by tulobuterol, that is, it is possible to prevent the medicated layer from having an excessively large adhesive strength, thereby eliminating the fear of increasing skin irritation.

Furthermore, by using the resolvent and the pressure-sensitive adhesive together for the adhesive skin patch of the present invention, it is possible not only to allow the medicated layer to have excellent skin adhesion properties for the skin but also to peel off the adhesive skin patch from the skin without skin irritation. In addition, it is also possible to reapply the adhesive skin patch onto the skin. Therefore, the adhesive skin patch of the present invention can be reliably applied onto a desired area of the skin and can be smoothly peeled off from the skin after use.

### BRIEF DESCRIPTION OF THE DRAWINGS

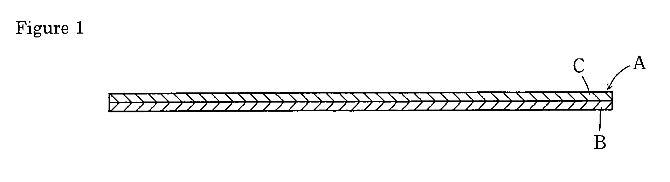

Fig. 1 is a vertical sectional view of an adhesive skin patch according to the present invention.

### EXPLANATION OF SYMBOLS

A: Adhesive skin patch
B: Base material
C: Medicated layer

### BEST MODE FOR CARRYING OUT THE INVENTION

(Solubility of tulobuterol in resolvents)
50 or 150 parts by weight of tulobuterol was added to each of the following resolvents in such a manner that the total amount of an obtained tulobuterol solution became 1000 parts by weight: 2-octyl-1-dodecanol (manufactured by Cognis Japan Ltd., under the trade name of "EUTANOL G"), 2-hexyl-1-decanol (manufactured by Cognis Japan Ltd., under the trade name of "EUTANOL G16N"), isostearyl alcohol (manufactured by Kokyu Alcohol Kogyo Co., Ltd., under the trade name of "Isostearyl Alcohol EX"), oleyl alcohol (manufactured by Kokyu Alcohol Kogyo Co., Ltd., under the trade name of "Oleyl Alcohol VP"), cetanol (hexadecyl alcohol) (manufactured by NOF Corporation, under the trade name of "NAA-44"), isopropyl myristate (manufactured by Nikko Chemicals Co., Ltd., under the trade name of "IPM-100"), decyl oleate (manufactured by Cognis Japan Ltd., under the trade name of "CETIOL V"), medium chain triglyceride (manufactured by NOF Corporation, under the trade name of "PANACET 800"), polyethylene glycol (manufactured by Maruishi Pharmaceutical Co., Ltd., under the trade name of "MACROGOL 400 (Japanese Pharmacopoeia)"), propylene glycol (manufactured by Maruishi Pharmaceutical Co., Ltd., under the trade name of "Propylene Glycol (Japanese Pharmacopoeia)"), and liquid paraffin (manufactured by Sankei Sangyo K.K., under the trade name of "Liquid Paraffin No. 350"). Each of the thus obtained tulobuterol solutions was kept at 50°C for 120 minutes, and was then kept at 20°C for 24 hours, and was further kept at 4°C for 24 hours.

At this time, the tulobuterol solution was visually observed to see whether crystalline tulobuterol was deposited or not after the completion of each warming of the tulobuterol solution at 50, 20 or 4°C. The results are shown in Table 1 wherein the mark "○" represents a case where crystalline tulobuterol was not observed, and the mark "×" represents a case where crystalline tulobuterol was observed. It is to be noted that in a case where tulobuterol was added to cetanol, tulobuterol was completely dissolved in cetanol when the tulobuterol solution was kept at 50°C, but it was impossible to check the presence or absence of crystalline tulobuterol when the tulobuterol solution was kept at 20°C or 4°C because cetanol was solidified.

(Solubility of epinephrine, levodopa butyl ester, phenacetin or phenylalanine in resolvents)
50 or 150 parts by weight of epinephrine (manufactured by Wako Pure Chemical Industries, Ltd.), levodopa butyl ester, phenacetin (manufactured by Wako Pure Chemical Industries, Ltd.) or phenylalanine (manufactured by Wako Pure Chemical Industries, Ltd.) was added to each of the above-mentioned resolvents (i.e., 2-octyl-1-dodecanol, 2-hexyl-1-decanol, isostearyl alcohol, oleyl alcohol, isopropyl myristate, polyethylene glycol and propylene glycol) in such a manner that the total amount of an obtained drug solution became 1,000 parts by weight. The solubility of each of these drugs in these resolvents was determined in the same manner as in a case where the solubility of tulobuterol in these resolvents was determined. The results are shown in Tables 2 to 5.

It is to be noted that levodopa butyl ester was produced by a method described in "J. Am. Chem. Soc., 75,5556-5560 (1953)". More specifically, 20.7 g of levodopa (manufactured by Sankyo Chemical Industries, Ltd.) was suspended in 360 mL of anhydrous 1-butanol (manufactured by Wako Pure Chemical Industries, Ltd.), and was then saturated by cooling with ice. The suspension was stirred for 1 hour, and was then heated under reflux.

Then, the anhydrous 1-butanol remaining in the reaction solution was removed under a reduced pressure. The residue thus obtained was dissolved in 900 mL of purified water to obtain an aqueous solution. Ammonia water was added to the aqueous solution for basification, and was then left at rest to deposit crystalline levodopa butyl ester. The crystalline levodopa butyl ester thus obtained was collected by filtration, and was then washed with hexane and ethyl acetate. Thereafter, levodopa butyl ester was recrystallized using ethyl acetate. The structure of the levodopa butyl ester thus obtained was determined using NMR.

(Solubility of tyramine in resolvents)
50 or 150 parts by weight of tyramine (manufactured by Tokyo Chemical Industries Co., Ltd.) was added to each of the above-mentioned resolvents (i.e., 2-octyl-1-dodecanol, isopropyl myristate, polyethylene glycol, and propylene glycol) in such a manner that the total amount of an obtained tyramine solution became 1000 parts by weight. The solubility of tyramine in these resolvents was determined in the same manner as in a case where the solubility of tulobuterol in these resolvents was determined. The results are shown in Table 6.

As can be seen from the results of solubility of each of the drugs in these resolvents, tulobuterol was stably dissolved in 2-octyl-1-dodecanol, 2-hexyl-1-decanol, isostearyl alcohol or oleyl alcohol within a wide temperature range of 4 to 50°C.

On the other hand, epinephrine, levodopa butyl ester, phenacetin, phenylalanine, and tyramine were not stably dissolved in 2-octyl-1-dodecanol, 2-hexyl-1-decanol, isostearyl alcohol or oleyl alcohol used for the adhesive skin patch of the present invention, and were deposited at any temperature within a temperature range of 4 to 50°C.

(Production of pressure-sensitive adhesive A)
2286 parts by weight of dodecyl methacrylate, 14256 parts by weight of 2-ethylhexyl methacrylate, 1656 parts by weight of 2-ethylhexyl acrylate, 2.3 parts by weight of hexanediol dimethacrylate, and 8500 parts by weight of ethyl acetate were fed into a 40-liter polymerization reactor, and were then heated to 80°C in an atmosphere of nitrogen.

Then, a solution obtained by dissolving 16 parts by weight of lauroyl peroxide in 1500 parts by weight of cyclohexane was gradually added to the reaction liquid contained in the polymerization reactor over 24 hours for polymerization. Ethyl acetate was further added to the reaction liquid to obtain a pressure-sensitive adhesive A having a solid concentration of 35% by weight.

(Production of pressure-sensitive adhesive solution B)
120 parts by weight of 2-ethylhexyl methacrylate, 60 parts by weight of ethyl acrylate, 20 parts by weight of vinyl pyrrolidone, 0.04 part by weight of trimethylol propane trimethacrylate, and 200 parts by weight of ethyl acetate were fed into a separable flask, and were then heated to 80°C in an atmosphere of nitrogen.

Then, a solution obtained by dissolving 1 part by weight of lauroyl peroxide in 100 parts by weight of cyclohexane was gradually added to the reaction liquid contained in the separable flask over 32 hours for polymerization. Ethyl acetate was further added to the reaction liquid to obtain a pressure-sensitive adhesive solution B having a solid concentration of 32% by weight.

(Examples 1 to 8, Comparative Examples 1 to 11)
In each of Examples 1 to 8 and Comparative Examples 1 to 11, the pressure-sensitive adhesive solution, the resolvent, and the drug shown in Table 7 were mixed in such a manner that the weight ratio among the pressure-sensitive adhesive, the resolvent, and the drug contained in the medicated layer became a value shown in Table 7. Then, ethyl acetate was added to the mixture to dilute the mixture in such a manner that the mixture had a solid concentration of 25% by weight, and then they were homogeneously mixed to obtain a coating liquid.

Thereafter, a polyethylene terephthalate film having one silicone-treated surface and a thickness of 38 µm was prepared. The coating liquid was applied onto the silicone-treated surface of the polyethylene terephthalate film, and was then dried at 60°C for 30 minutes to remove ethyl acetate and cyclohexane. In this way, a medicated layer having a thickness shown in Table 7 was formed on one surface of the polyethylene terephthalate film. Then, a polyethylene terephthalate film having a thickness of 38 µm was prepared as a base material. The base material was integrally laminated on the medicated layer to obtain an adhesive skin patch (I).

An adhesive skin patch (II) was manufactured in the same manner as in the case of the adhesive skin patch (I) except that the polyethylene terephthalate film as a base material was replaced with a base material obtained by integrally laminating a polyethylene terephthalate film having a thickness of 12 µm on a polyester fiber non-woven fabric having a weight of 40 g/m² and that the polyethylene terephthalate film of the base material was integrally laminated on the medicated layer.

The tulobuterol deposition properties, tackiness, skin irritating properties, skin adhesion properties, and permeability of these adhesive skin patches were determined in the following manner. The results are shown in Table 8.

(Tulobuterol deposition properties)
Just after the manufacture of the adhesive skin patch (I), two planar square-shaped specimens having a size of 50 mm × 50 mm were cut out from the adhesive skin patch (I). Each of the specimens was covered with an aluminum foil, and was then hermetically sealed in an aluminum packing material. Then, one specimen was left standing in a thermostatic bath set at 25°C and the other specimen was left standing in a thermostatic bath set at 4°C.

Just after the specimens were cut out from the adhesive skin patch (I), and after the lapse of one month and three months, respectively, from the time when the specimens were placed in the thermostatic bathes, the medicated layer surface of each of the specimens was observed with an optical microscope to see whether crystalline tulobuterol was deposited on the medicated layer surface or not. In Table 8, the mark "○" represents a case where crystalline tulobuterol was not deposited, and the mark "×" represents a case where crystalline tulobuterol was deposited. It is to be noted that in Comparative Example 3, a deposited crystal was considered as crystalline cetanol.

(Tackiness)
Planar rectangle-shaped specimens having a size of 50 mm long and 15 mm wide were cut out from the adhesive skin patch (I), and a 180° peel test was carried out three times according to a testing method for adhesive strength defined in JIS Z0237. The tackiness of the adhesive skin patch was evaluated based on the arithmetic mean of measured adhesive strength values.

(Skin irritating properties)
Six planar square-shaped specimens having a size of 2 cm × 2 cm were cut out from the adhesive skin patch (II). Rabbits were prepared, and the dorsal skin thereof was shaved with a hair clipper and a shaver. The specimens were applied to the pre-shaved dorsal skin of the rabbits for 24 hours, and were then peeled off from the skin. After the lapse of 30 minutes and 24 hours, respectively, from the time when the specimens were peeled off from the skin, the skin was visually observed to check the level of erythema according to the following criteria. The skin irritating properties of the adhesive skin patch (II) were evaluated based on the arithmetic mean of evaluation scores of the specimens. It is to be noted that in Comparative Example 11, evaluations could not be made because a lot of adhesive residue was left on the skin.
0 No erythema was observed.
1 Very mild erythema was observed (erythema was barely able to see).
2 Erythema was clearly observed.
3 Moderate to severe erythema was observed.
4 Severe erythema of a deep red color was observed and a small scab was formed.

(Skin adhesion properties and presence/absence of adhesive residue)
After the lapse of 24 hours from the time when the specimens were applied to the dorsal skin of the rabbits for the test of skin irritating properties, the area of part of the medicated layer of each of the specimens that was still adhered to the dorsal skin of the rabbit without peeling was measured. The skin adhesion properties were evaluated based on the percentage of the thus measured area with respect to the entire area of the medicated layer.

Further, the skin surface of each of the rabbits was visually observed after the completion of the test of skin irritating properties, and the presence or absence of an adhesive residue on the skin was evaluated according to the following criteria.
○ ... An adhesive residue was hardly observed.
Δ ... An adhesive residue was observed in an area of the skin where the outer peripheral portion of the specimen had been adhered.
× ... An adhesive residue was observed in the entire area of the skin where the specimen had been adhered.
×× Cohesive failure was observed in the entire area of the skin where the specimen had been adhered.

(Permeability)
Planar circle-shaped specimens having a diameter of 2 cm (area: 3.14 cm²) were cut out from each of the adhesive skin patches (I) of Examples 1 to 8 and Comparative Examples 1 to 8 and 10. At the same time, dorsal skin was excised from a hairless mouse (male, 8-week old), and was then fixed to a Franz diffusion cell maintained at 37°C. The medicated layer of the specimen was applied to the upper end portion of the skin. A normal saline solution adjusted to pH 7.2 was used as a receptor fluid, and the lower end portion of the skin was immersed in the receptor fluid.

After the lapse of 4, 8, 21, and 24 hours, respectively, from the time when the specimen was applied to the skin, the receptor fluid in which the lower portion of the skin was immersed was sampled to measure the concentration of tulobuterol by HPLC. It is to be noted that three specimens were prepared for each of the adhesive skin patches, and the tulobuterol concentration of the receptor fluid was measured for each of the specimens in such a manner described above after the lapse of 4, 8, 21, and 24 hours, respectively. The amount of tulobuterol permeated through the skin was calculated from the tulobuterol concentration of the receptor fluid and the amount of the receptor fluid after the lapse of 4, 8, 21, and 24 hours, respectively. The arithmetic mean of the tulobuterol permeation amounts calculated for the three specimens was determined after the lapse of 4, 8, 21, and 24 hours, respectively, and the arithmetic mean thus determined was defined as a cumulative skin permeation amount. It is to be noted that when the tulobuterol permeation amount was calculated after the lapse of 8, 21, and 24 hours, respectively, correction was made to the sample volume of the receptor fluid because the receptor fluid had already been sampled.

A time period of 4 hours from the time when the specimen was applied to the skin was defined as "Period 1", a time period of 4 hours from the end of Period 1 was defined as "Period 2", a time period of 13 hours from the end of the Period 2 was defined as "Period 3", and a time period of 3 hours from the end of the Period 3 was defined as "Period 4". The average rate of absorption of tulobuterol was calculated by dividing the tulobuterol permeation amount increased within each time period by hours in each time period. As a result, in a case where the adhesive skin patches of Examples 1, 2, 5, and 6 were used, the rate of absorption of tulobuterol was not greatly changed from Period 1 to Period 4, which indicates that tulobuterol was stably absorbed through the skin.

**Table 1**

| Solubility of tulobuterol in resolvents | | | | | | |
|---|---|---|---|---|---|---|
| Resolvents | 50°C | | 20°C | | 4°C | |
| | 50 parts by weight | 150 parts by weight | 50 parts by weight | 150 parts by weight | 50 parts by weight | 150 parts by weight |
| 2-octyl-1-dodecanol | ○ | ○ | ○ | ○ | ○ | ○ |
| 2-hexyl-1-decanol | ○ | ○ | ○ | ○ | ○ | ○ |
| Isostearyl alcohol | ○ | ○ | ○ | ○ | ○ | ○ |
| Oleyl alcohol | ○ | ○ | ○ | ○ | ○ | ○ |
| Cetanol | ○ | ○ | - | - | - | - |
| Isopropyl myristate | ○ | ○ | ○ | × | ○ | × |
| Decyl oleate | ○ | × | ○ | × | ○ | × |
| Medium chain triglyceride | ○ | × | ○ | × | ○ | × |
| Polyethylene glycol | ○ | × | ○ | × | ○ | × |
| Propylene glycol | ○ | ○ | ○ | × | ○ | × |
| Liquid paraffin | × | × | × | × | × | × |

**Table 2**

| Solubility of epinephrine in resolvents | | | | | | |
|---|---|---|---|---|---|---|
| Resolvents | 50°C | | 20°C | | 4°C | |
| | 50 parts by weight | 150 parts by weight | 50 parts by weight | 150 parts by weight | 50 parts by weight | 150 parts by weight |
| 2-octyl-1-dodecanol | × | × | × | × | × | × |
| 2-hexyl-1-decanol | × | × | × | × | × | × |
| Isostearyl alcohol | × | × | × | × | × | × |
| Oleyl alcohol | × | × | × | × | × | × |
| Isopropyl myristate | × | × | × | × | × | × |
| Polyethylene glycol | × | × | × | × | × | × |
| Propylene glycol | × | × | × | × | × | × |

**Table 3**

| Solubility of levodopa butyl ester in resolvents | | | | | | |
|---|---|---|---|---|---|---|
| Resolvents | 50°C | | 20°C | | 4°C | |
| | 50 parts by weight | 150 parts by weight | 50 parts by weight | 150 parts by weight | 50 parts by weight | 150 parts by weight |
| 2-octyl-1-dodecanol | × | × | × | × | × | × |
| 2-hexyl-1-decanol | × | × | × | × | × | × |
| Isostearyl alcohol | × | × | × | × | × | × |
| Oleyl alcohol | × | × | × | × | × | × |
| Isopropyl myristate | × | × | × | × | × | × |
| Polyethylene glycol | ○ | × | ○ | × | ○ | × |
| Propylene glycol | ○ | ○ | ○ | × | ○ | × |

**Table 4**

| Solubility of phenacetin in resolvents | | | | | | |
|---|---|---|---|---|---|---|
| Resolvents | 50°C | | 20°C | | 4°C | |
| | 50 parts by weight | 150 parts by weight | 50 parts by weight | 150 parts by weight | 50 parts by weight | 150 parts by weight |
| 2-octyl-1-dodecanol | × | × | × | × | × | × |
| 2-hexyl-1-decanol | × | × | × | × | × | × |
| Isostearyl alcohol | × | × | × | × | × | × |
| Oleyl alcohol | × | × | × | × | × | × |
| Isopropyl myristate | × | × | × | × | × | × |
| Polyethylene glycol | ○ | × | × | × | × | × |
| Propylene glycol | × | × | × | × | × | × |

**Table 5**

| Solubility of phenylalanine in resolvents | | | | | | |
|---|---|---|---|---|---|---|
| Resolvents | 50°C | | 20°C | | 4°C | |
| | 50 parts by weight | 150 parts by weight | 50 parts by weight | 150 parts by weight | 50 parts by weight | 150 parts by weight |
| 2-octyl-1-dodecanol | × | × | × | × | × | × |
| 2-hexyl-1-decanol | × | × | × | × | × | × |
| Isostearyl alcohol | × | × | × | × | × | × |
| Oleyl alcohol | × | × | × | × | × | × |
| Isopropyl myristate | × | × | × | × | × | × |
| Polyethylene glycol | × | × | × | × | × | × |
| Propylene glycol | × | × | × | × | × | × |

**Table 6**

| Solubility of tyramine in resolvents | | | | | | |
|---|---|---|---|---|---|---|
| Resolvents | 50°C | | 20°C | | 4°C | |
| | 50 parts by weight | 150 parts by weight | 50 parts by weight | 150 parts by weight | 50 parts by weight | 150 parts by weight |
| 2-octyl-1-dodecanol | × | × | × | × | × | × |
| Isopropyl myristate | × | × | × | × | × | × |
| Polyethylene glycol | × | × | × | × | × | × |
| Propylene glycol | × | × | × | × | × | × |

**Table 7**

| | Pressure sensitive adhesive | | Resolvent | | Drug (wt%) | | Thickness of medicated layer (µm) |
|---|---|---|---|---|---|---|---|
| | Type | Amount (parts by weight) | Type | Amount (parts by weight) | Type | Amount (parts by weight) | |
| Example 1 | A | 100 | 2-octyl-1-dodecanol | 11.5 | Tulobuterol | 3.4 | 80 |
| Example 2 | A | 100 | Oleyl alcohol | 5.4 | Tulobuterol | 3.3 | 80 |
| Example 3 | A | 100 | 2-hexyl-1-decanol | 5.4 | Tulobuterol | 3.3 | 80 |
| Example 4 | A | 100 | 2-octyl-1-dodecanol | 18.5 | Tulobuterol | 4.9 | 120 |
| Example 5 | A | 100 | 2-octyl-1-dodecanol | 33.3 | Tulobuterol | 5.6 | 120 |
| Example 6 | A | 100 | Oleyl alcohol | 11.6 | Tulobuterol | 4.7 | 120 |
| Example 7 | A | 100 | Oleyl alcohol | 27 | Tulobuterol | 8.1 | 120 |
| Example 8 | A | 100 | 2-octyl-1-dodecanol | 11.5 | Tulobuterol | 16.7 | 80 |
| Comparative Example 1 | A | 100 | - | - | Tulobuterol | 3.1 | 80 |
| Comparative Example 2 | A | 100 | Cetanol | 2.6 | Tulobuterol | 3.2 | 80 |
| Comparative Example 3 | A | 100 | Cetanol | 5.4 | Tulobuterol | 3.3 | 80 |
| Comparative Example 4 | A | 100 | Decyl oleate | 5.4 | Tulobuterol | 3.3 | 80 |
| Comparative Example 5 | A | 100 | Isopropyl myristate | 5.4 | Tulobuterol | 3.3 | 80 |
| Comparative Example G | A | 100 | Isopropyl myristate | 18.3 | Tulobuterol | 3.7 | 80 |
| Comparative Example 7 | B | 100 | - | - | Tulobuterol | 3.1 | 80 |
| Comparative Example 8 | B | 100 | 2-octyl-1-dodecanol | 11.5 | Tulobuterol | 3.4 | 80 |
| Comparative Example 9 | A | 100 | 2-octyl-1-dodecanol | 11.5 | Levodopa | 3.4 | 80 |
| Comparative Example 10 | A | 100 | - | - | Tulobuterol | 13.6 | 80 |
| Comparative Example 11 | A | 100 | 2-octyl-1-dodecanol | 76.4 | Tulobuterol | 5.5 | 80 |

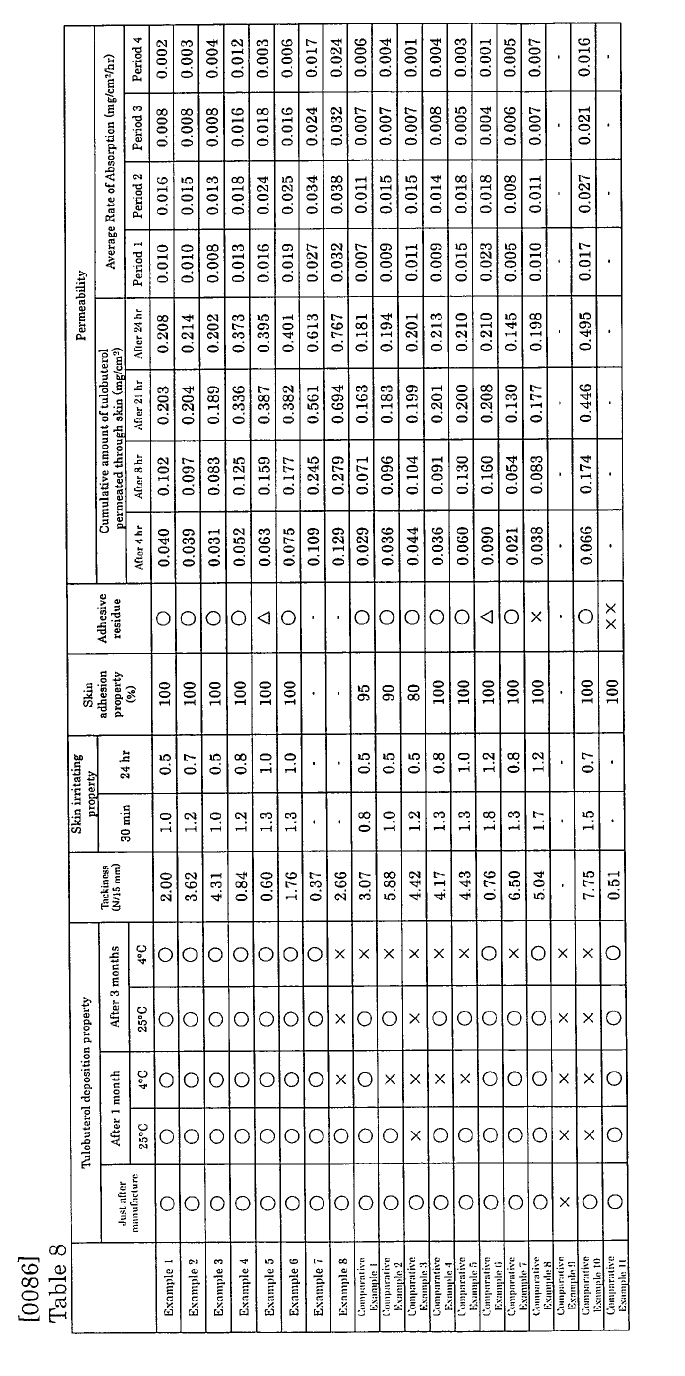

### INDUSTRIAL APPLICABILITY

The adhesive skin patch according to the present invention is a percutaneous absorption preparation of tulobuterol. More specifically, this adhesive skin patch permits prolonged percutaneous absorption of tulobuterol and can be stably applied to the skin for a long period of time with less skin irritation.

## Claims

1. An adhesive skin patch comprising: a base material; and a medicated layer provided on one surface of the base material and containing 100 parts by weight of a pressure-sensitive adhesive, 1 to 75 parts by weight of a resolvent, and tulobuterol, the resolvent containing an aliphatic alcohol having a branched-chain structure or a double bond in its C8 to C30 carbon chain, and the pressure-sensitive adhesive being a copolymer obtained by copolymerizing monomers containing 70% by weight or more of an alkyl(meth)acrylate whose alkyl group has 6 to 20 carbon atoms.

2. The adhesive skin patch according to claim 1, wherein the medicated layer contains 100 parts by weight of the pressure-sensitive adhesive, 1 to 75 parts by weight of the resolvent, and 0.5 to 75 parts by weight of tulobuterol.

3. The adhesive skin patch according to claim 1, wherein the medicated layer contains 100 parts by weight of the pressure-sensitive adhesive, 1 to 75 parts by weight of the resolvent, and 1 to 25 parts by weight of tulobuterol.

4. The adhesive skin patch according to claim 1, wherein the medicated layer contains 100 parts by weight of the pressure-sensitive adhesive, 1 to 40 parts by weight of the resolvent, and 1 to 25 parts by weight of tulobuterol.

5. The adhesive skin patch according to claim 1, wherein the medicated layer contains 100 parts by weight of the pressure-sensitive adhesive, 2 to 40 parts by weight of the resolvent, and 2 to 11 parts by weight of tulobuterol.

6. The adhesive skin patch according to claim 1, wherein the medicated layer contains 100 parts by weight of the pressure-sensitive adhesive, 2 to 30 parts by weight of the resolvent, and 2 to 11 parts by weight of tulobuterol.

7. The adhesive skin patch according to claim 1, wherein the resolvent is an aliphatic alcohol having a branched-chain structure in its C8 to C30 carbon chain.

8. The adhesive skin patch according to claim 1, wherein the resolvent is an aliphatic alcohol having a branched-chain structure in its C12 to C24 carbon chain.

9. The adhesive skin patch according to claim 1, wherein the resolvent is an aliphatic alcohol having a double bond in its C12 to C24 carbon chain.

10. The adhesive skin patch according to claim 1, wherein the resolvent is octyl dodecanol.

11. The adhesive skin patch according to claim 1, wherein the pressure-sensitive adhesive is a copolymer obtained by copolymerizing monomers containing 70% by weight or more of 2-ethylhexyl(meth)acrylate.

12. The adhesive skin patch according to claim 1, wherein the pressure-sensitive adhesive is a copolymer obtained by copolymerizing monomers containing 2-ethylhexyl(meth)acrylate and an alkyl(meth)acrylate whose alkyl group is linear and has 6 to 20 carbon atoms.

13. The adhesive skin patch according to claim 12, wherein the monomers contain 70 to 95% by weight of 2-ethylhexyl(meth)acrylate.

14. The adhesive skin patch according to claim 12, wherein the monomers contain 5 to 30% by weight of an alkyl(meth)acrylate whose alkyl group is linear and has 6 to 20 carbon atoms.

15. The adhesive skin patch according to claim 12, wherein the monomers contain 70 to 95% by weight of 2-ethylhexyl(meth)acrylate and 5 to 30% by weight of an alkyl(meth)acrylate whose alkyl group is linear and has 6 to 20 carbon atoms.
